(19) Europäisches Patentamt
European Patent Office
Office européen des brevets

(11) Numéro de publication: **0 002 156**
**B1**

# (12) FASCICULE DE BREVET EUROPEEN

(45) Date de publication du fascicule de brevet: **13.05.81**

(21) Numéro de dépôt: **78400179.4**

(22) Date de dépôt: **14.11.78**

(51) Int. Cl.[3]: **C 07 C 93/12,** C 07 C 93/14, A 61 K 31/135, A 61 K 31/04 // C07C91/14, C07C97/07, C07C125/06

(54) **Nouveaux dérivés de la 5H-benzocyclohepten-7 amine et leurs sels, leur procédé de préparation, leur application à titre de médicaments et les compositions pharmaceutiques les renfermant.**

(30) Priorité: **18.11.77 FR 7734738**

(43) Date de publication de la demande: **30.05.79 Bulletin 79/11**

(45) Mention de la délivrance du brevet: **13.05.81 Bulletin 81/19**

(84) Etats contractants désignés: **BE CH DE FR GB LU NL SE**

(56) Documents cités: **FR-A-2 319 332**

(73) Titulaire: **ROUSSEL-UCLAF, 102, route de Noisy Boîte postale no.9, F-93230-Romainville (FR)**

(72) Inventeur: **Nedelec, Lucien, 45 boulevard de l'Ouest, F-93340-Le Raincy (FR)**
Inventeur: **Frechet, Daniel, 45 rue Lecourbe, F-75015 Paris (FR)**
Inventeur: **Dumont, Claude, 33 rue du Maréchal Vaillant, F-94130 Nogent sur Marne (FR)**
Inventeur: **Hunt, Peter, 6 avenue du Lycée, F-95500 Gonesse (FR)**

(74) Mandataire: **Vieillefosse, Jean-Claude et al, boîte postale no 9 102, route de Noisy, F-93230 Romainville (FR)**

**EP 0 002 156 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

# Nouveaux dérivés de la 5H-benzocyclohepten-7-amine et leurs sels, leur procédé de préparation, leur application à titre de médicaments et les compositions pharmaceutiques les renfermant

La présente invention concerne de nouveaux dérivés de la 5H-benzocyclohepten-7-amine et leurs sels, leur procédé de préparation, leur application à titre de médicaments et les compositions pharmaceutiques les renfermant.

L'invention a pour objet de nouveaux dérivés de la 5H-benzocyclohepten-7-amine, ainsi que leurs sels d'addition avec les acides minéraux ou organiques, caractérisés en ce qu'ils répondent à la formule générale:

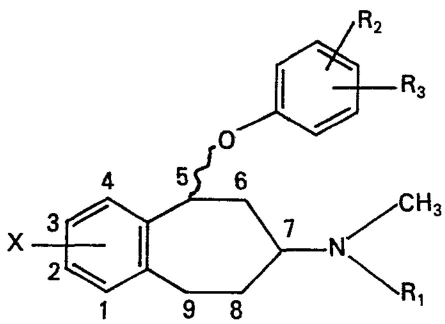

(I')

dans laquelle $R_1$ représente un atome d'hydrogène ou un radical méthyle, $R_2$ et $R_3$, identiques ou différents, représentent un atome d'hydrogène, un atome de fluor, de chlore ou de brome, un radical nitro, amino, trifluorométhyle ou méthoxy et X représente un atome d'hydrogène ou un atome d'halogène.

Parmi les composés de formule I', l'invention a notamment pour objet les composés de formule générale:

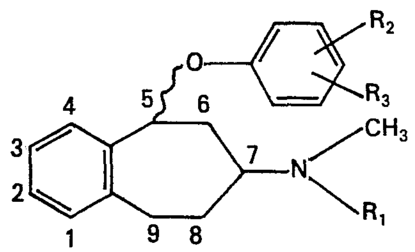

(I)

dans laquelle $R_1$, $R_2$ et $R_3$ sont définis comme ci-dessus.

Dans la formule (I') ou (I) et dans ce qui suit, le trait ondulé en position 5 indique la présence d'isomères cis ou trans.

Les sels d'addition avec les acides minéraux ou organiques peuvent être, par exemple, les sels formés avec les acides chlorhydrique, bromhydrique, iodhydrique, nitrique, sulfurique, phosphorique, acétique, formique, benzoïque, maléique, fumarique, succinique, tartrique, citrique, oxalique, glyoxylique, aspartique, alcanesulfoniques tels que les acides méthane ou éthanesulfoniques, arylsulfoniques tels que les acides benzène ou paratoluène sulfoniques et arylcarboxyliques.

Parmi les produits, objet de l'invention, on peut citer notamment les dérivés répondant à la formule (I) ci-dessus, ainsi que leurs sels d'addition avec les acides minéraux ou organiques, caractérisés en ce que dans ladite formule (I), $R_1$ représente un radical méthyle et $R_2$ et $R_3$ ont la signification déjà indiquée.

Parmi ces derniers, on peut citer les dérivés répondant à la formule (I) ci-dessus, ainsi que leurs sels d'addition avec les acides minéraux ou organiques, caractérisés en ce que dans ladite formule (I), l'un au moins de $R_2$ et $R_3$ représente un atome d'hydrogène.

Parmi les produits, objet de l'invention, on peut citer plus particulièrement:

- les isomères cis et trans de la N,N-diméthyl 5-[(4-nitrophényl)oxy] 6,7,8,9-tétrahydro 5H-benzocyclohepten-7-amine,
- l'isomère cis de la N,N-diméthyl 6,7,8,9-tétrahydro 5-[[4-trifluorométhyl)phényl]oxy] 5H-benzocyclohepten-7-amine,
- l'isomère cis de la N,N-diméthyl 5-[(4-bromophényl)oxy] 6,7,8,9-tétrahydro 5H-benzocyclohepten-7-amine, ainsi que leurs sels d'addition avec les acides minéraux ou organiques.

L'invention a également pour objet un procédé de préparation des dérivés définis par la formule (I') ci-dessus et notamment des dérivés définis par la formule (I), ainsi que de leurs sels, ledit procédé étant caractérisé en ce que l'on fait réagir un produit de formule:

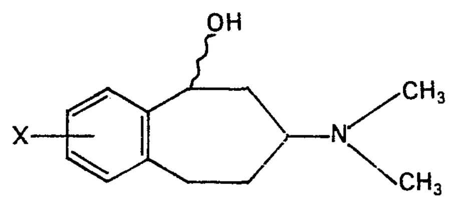

(II)

dans laquelle X est défini comme précédemment, avec un produit de formule:

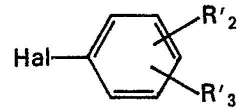

(III)

dans laquelle Hal représente un atome de fluor, de chlore ou de brome, $R'_2$ représente un atome de fluor, de chlore ou de brome, un radical nitro, trifluorométhyle ou méthoxy et $R'_3$ représente un atome d'hydrogène ou $R'_2$, pour obtenir un produit de formule:

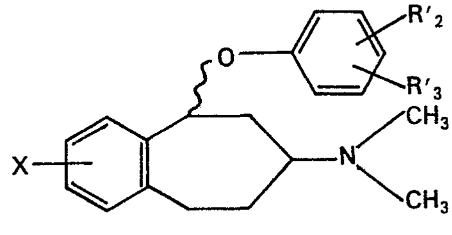

($I_A$)

dans laquelle X, $R'_2$ et $R'_3$ ont la signification déjà indiquée et que,

- soit, à condition d'avoir préparé un produit de formule ($I_A$) dans laquelle l'un au moins de $R'_2$ ou de $R'_3$ représente un radical nitro, on réduit ledit produit de formule ($I_A$) pour obtenir un produit de formule:

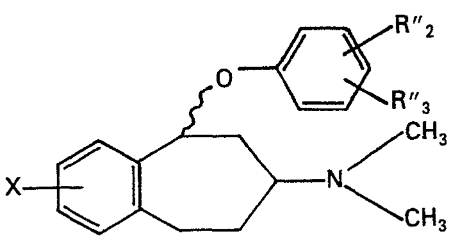

($I_B$)

dans laquelle X a la signification déjà indiquée, l'un au moins de $R''_2$ ou de $R''_3$ représente un radical amino et l'autre substituant $R''_2$ ou $R''_3$ a la signification de $R_2$,

- soit, à condition d'avoir préparé un produit de formule ($I_A$) dans laquelle $R'_2$ représente un atome de brome et $R'_3$ représente un atome d'hydrogène, un hydrogénolyse ledit prodit de formule ($I_A$) pour obtenir un produit de formule:

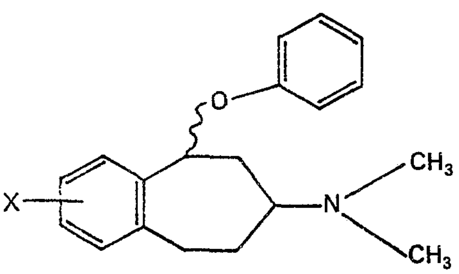

($I_C$)

dans laquelle X a la signification déjà indiquée et que, soit on isole et, si désiré, salifie les produits de formule ($I_A$), ($I_B$), ($I_C$) ainsi obtenus, correspondant à des produits de formule (I') dans laquelle $R_1$ représente un radical méthyle, soit enfin on désalcoyle ces derniers et, le cas échéant, salifie les produits de formule (I') ainsi obtenus dans laquelle X, $R_2$ et $R_3$ ont la signification déjà indiquée et $R_1$ représente un atome d'hydrogène.

L'isomérie des produits est conservée au cours du procédé de préparation. C'est ainsi que lorsque l'on utilise comme produit de départ de formule (II), l'isomère cis, on obtient le produit de formule (I') sous forme d'isomère cis. Il en est de même pour l'isomère trans.

Dans des conditions préférentielles de mise en œuvre de l'invention, le procédé de préparation ci-dessus décrit est caractérisé en ce que:

a) la réaction du produit de formule (II) avec le produit de formule (III) est effectuée en présence d'hydrure de sodium dans le tétrahydrofuranne ou de préférence dans le diméthylsulfoxyde,

b) la réduction du produit de formule ($I_A$) en produit de formule ($I_B$) est effectuée au moyen de l'hydrogène en présence d'un catalyseur tel que le bioxyde de platine au sein d'un alcanol de bas poids moléculaire tel que l'éthanol,

c) l'hydrogénolyse du produit de formule ($I_A$) en produit de formule ($I_C$) est effectuée au moyen de l'hydrogène, en présence d'un catalyseur tel que le palladium, au sein d'un alcanol de bas poids moléculaire tel que l'éthanol,

d) la désalcoylation des produits de formule (I') est effectuée par l'intermédiaire d'un halogénoformiate d'alcolyle tel que le chloroformiate d'éthyle, de préférence au sein d'un solvant organique tel que le benzène, ou au moyen de l'azodicarboxylate d'éthyle et est suivie par une hydrolyse acide ou alcaline.

Les dérivés de formule (I') présentent un caractère basique. On peut avantageusement préparer les sels d'addition desdits dérivés en faisant réagir ces derniers avec un acide minéral ou organique en proportions sensiblement stœchiométriques.

Les produits, objet de la présente invention, possédent de très intéressantes propriétés pharmacologiques; ils sont doués notamment de remarquables propriétés inhibitrices sur la captation de la sérotonine, in vitro et in vivo.

Certains produits et notamment le fumarate de N,N-diméthyl 5-[(4-nitrophényl)oxy] 6,7,8,9-tétrahydro 5H-benzocyclohepten-7-amine (cis) possèdent, également, d'intéressantes propriétés anorexigènes.

Ces propriétés sont illustrées plus loin dans la partie expérimentale.

Ces propriétés justifient l'utilisation des dérivés de la 5H-benzocyclohepten-7-amine selon l'invention et de leurs sels, à titre de médicaments.

La présente demande a ainsi également pour objet l'application à titre de médicaments des dérivés de la 5H-benzocyclohepten-7-amine tels que définis par la formule (I') ainsi que des sels d'addition avec les acides pharmaceutiquement acceptables desdits dérivés.

Parmi les médicaments, selon l'invention, on retient de préférence les nouveaux dérivés de la 5H-benzocyclohepten-7-amine répondant à la formule (I) dans laquelle $R_1$ représente un radical méthyle et $R_2$ et $R_3$ ont la signification déjà indiquée ainsi que leurs sels d'addition avec les acides pharmaceutiquement acceptables.

Parmi ceux-ci, on retient notamment ceux répondant à la formule (I) dans laquelle l'un au moins de $R_2$ ou $R_3$ représente un atome d'hydrogène ainsi que leurs sels d'addition avec les acides pharmaceutiquement acceptables.

Parmi les médicaments selon l'invention, on retient enfin tout particulièrement les produits dont les noms suivent:

- les isomères cis et trans de la N,N-diméthyl 5-[(4-nitrophényl)oxy] 6,7,8,9-tétrahydro 5H-benzocyclohepten-7-amine,
- l'isomère cis de la N,N-diméthyl 6,7,8,9-tétrahydro 5-[[4-(trifluorométhyl)phényl]oxy] 5H-benzocyclohepten-7-amine,
- l'isomère cis de la N,N-diméthyl 5-[(4-bromophényl)-oxy] 6,7,8,9-tétrahydro 5H-benzocyclohepten-7-amine, ainsi que leurs sels d'addition avec les acides pharmaceutiquement acceptables.

Ces médicaments trouvent par exemple leur emploi dans le traitement des dépressions, de la mélancolie, des psychoses maniaco-dépressives,

des dépressions réactionnelles et d'épuisement, des dépressions névrotiques.

Ceux doués de propriétés anorexigènes peuvent, en outre, être utilisées «dans le traitement de l'obésité».

La dose usuelle, variable selon le produit utilisé, le sujet traité et l'affection en cause peut être par exemple de 10 mg à 300 mg par jour, par voie orale chez l'homme.

L'invention a enfin pour objet les compositions pharmaceutiques qui renferment au moins un dérivé précité ou un de ses sels d'addition avec les acides pharmaceutiquement acceptables à titre de principe actif.

A titre de médicaments, les dérivés répondent à la formule I' et leurs sels d'addition avec les acides pharmaceutiquement acceptables, peuventêtre incorporés dans des compositions pharmaceutiques destinées à la voie digestive ou parentérale; ces compositions pharmaceutiques peuvent être, par exemple, solides ou liquides et se présenter sous les formes pharmaceutiques couramment utilisées en médecine humaine, comme par exemple, les comprimé, simples ou dragéifiés, les gélules, les granules, les suppositoires, les préparations injectables. Elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les produits de départ de formule (II) peuvent être préparés en opérant comme indiqué dans la demande française publiée sous le n° 2.319.332.

Les isomères cis et trans du 7-diméthylamino 6,7,8,9-tétrahydro 5H-benzocyclohepten-5-ol peuvent être séparés du mélange des deux isomères par cristallisation ou par chromatographie sur gel de silice.

Il va être donné maintenant, à titre non limitatif, des exemples de mise en œuvre de l'invention.

Exemple 1

Fumarate de N,N-diméthyl 5-[(4-nitrophényl)-oxy] 6,7,8,9-tétrahydro 5H-benzocyclohepten-7-amine (cis)

A 960 mg d'hydrure de sodium en dispersion à 50% dans l'huile, on ajoute à température ambiante, 10 cm³ de diméthylsulfoxyde.

On agite 20 minutes à 40-45°C, refroidit vers 25°C puis ajoute 4,1 g de 7-diméthylamino 6,7,8,9-tétrahydro [5H]-benzocyclohepten-5-ol (cis).

On agite encore dix minutes vers 25°C et ajoute 3,15 g de p-chloronitrobenzène, agite à nouveau, vingt minutes à température ambiante, reprend par du chlorure de méthylène, lave à l'eau, sèche sur sulfate de magnésium, filtre et distille à sec sous vide. On obtient 8,7 g de produit brut que l'on reprend par trois fois 100 cm³ d'acide chlorhydrique 0,5N, élimine la fraction neutre par trois fois 100 cm³ d'éther, réunit les phases aqueuses et alcalinise par 50 cm³ d'ammoniaque concentrée.

On extrait par du chlorure de méthylène, sèche sur sulfate de magnésium, filtre et distille à sec sous vide.

On obtient 7 g de N,N-diméthyl 5-[(4-nitrophényl)oxy] 6,7,8,9-tétrahydro 5H-benzocyclohepten-7-amine (cis) sous forme d'une huile brune.

Préparation du fumarate

On dissout partiellement 2,32 g d'acide fumarique dans 25 cm³ de méthanol, ajoute à la suspension ainsi obtenue la solution de 7 g de la base obtenue ci-dessus dans 50 cm³ d'isopropanol.

On chauffe jusqu'à dissolution puis concentre sous un léger vide. On laisse deux heures à température ambiante et essore. On recueille 7,4 g de fumarate de N,N-diméthyl 5-[(4-nitrophényl)oxy] 6,7,8,9-tétrahydro 5H-benzocyclohepten-7-amine (cis) sous forme de cristaux incolores. (F = 194°C).

Analyse:
$C_{19}H_{22}N_2O_3$, $C_4H_4O_4$ = 442,45

| | C % | H % | N % |
|---|---|---|---|
| Calculé: | 62,43 | 5,92 | 6,33 |
| Trouvé: | 62,2 | 6,1 | 6,1 |

Les isomères cis et trans du 7-diméthylamino 6,7,8,9-tétrahydro 5H-benzocyclohepten-5-ol peuvent être séparés du mélange des deux isomères en opérant comme suit:

On sépare par chromatographie sur gel de silice 27,2 g de 7-diméthylamino 6,7,8,9-tétrahydro [5H]-benzocyclohepten-5-ol (mélange des deux isomères) obtenus au départ du 8,9-dihydro 5H-benzocyclohepten-5-one en opérant comme indiqué dans la demande française publiée sous le n° 2.319.332.

On élue par un mélange d'acétate d'éthyle et de triéthylamine (8-2) et récupère, après élimination de l'éluant 8,8 g d'une première fraction de Rf = 0,20 correspondant à l'isomère trans et 14,5 g d'une seconde fraction de Rf = 0,10 correspondant à l'isomère cis.

a) Isomère cis

On reprend par un minimum d'éther isopropylique, les 14,5 g de la seconde fraction correspondant à l'isomère cis, essore et obtient 12 g de produit attendu. Pour l'analyse, on fait recristalliser le produit dans l'éther et obtient ainsi l'isomère cis sous forme de cristaux incolores fondant à 140°C.

Analyse:
$C_{13}H_{19}NO$ = 205,28

| | C % | H % | N % |
|---|---|---|---|
| Calculé: | 76,05 | 9,33 | 6,82 |
| Trouvé: | 76,1 | 9,6 | 6,8 |

b) Isomère trans

On reprend par un minimum d'éther isopropylique les 8,8 g de la première fraction correspondant à l'isomère trans, essore et obtient 8,1 g de produit attendu.

Pour l'analyse, on fait recristalliser le produit dans l'éther, et obtient ainsi l'isomère trans sous forme de cristaux incolores fondant à 125 °C.

Analyse:

$C_{13}H_{19}NO = 205,28$

| | C % | H % | N % |
|---|---|---|---|
| Calculé: | 76,05 | 9,33 | 6,82 |
| Trouvé: | 75,9 | 9,6 | 6,8 |

Exemple 2

Dichlorhydrate de 5-[(4-aminophényl)oxy] N,N-diméthyl 6,7,8,9-tétrahydro 5H-benzocyclohepten-7-amine (cis)

On dissout dans 200 cm³ d'éthanol 3,7 g de N,N-diméthyl 5-[(4-nitrophényl)oxy] 6,7,8,9-tétrahydro 5H-benzocyclohepten-7-amine (cis) obtenus à l'exemple 1 et ajoute 370 mg de bioxyde de platine.

On hydrogène sous agitation (un litre d'hydrogène en 30 minutes) filtre et distille à sec sous vide.

On obtient 3,6 g de 5-[(4-aminophényl)oxy] N,N-diméthyl 6,7,8,9-tétrahydro 5H-benzocyclohepten-7-amine (cis) sous forme d'une huile orange.

Préparation du dichlorhydrate

On dissout les 3,6 g obtenus ci-dessus dans 20 cm³ d'acétate d'éthyle, ajoute une solution saturée d'acide chlorhydrique gazeux dans l'acétate d'éthyle, jusqu'à pH 1, laisse une heure à température ambiante et essore. On obtient 4,1 g du chlorhydrate attendu sous forme de cristaux incolores fondant à ≃ 260 °C.

Analyse:

$C_{19}H_{24}N_2O, 2HCl = 369,33$

| | C % | H % | Cl % | N % |
|---|---|---|---|---|
| Calculé: | 61,78 | 7,10 | 19,20 | 7,59 |
| Trouvé: | 61,5 | 7,1 | 19,2 | 7,6 |

Exemple 3

Chlorhydrate de N,N-diméthyl 6,7,8,9-tétrahydro 5-[[4-(trifluorométhyl)phényl]oxy] 5H-benzocyclohepten-7-amine (cis)

A 2 g d'hydrure de sodium en dispersion à 50% dans l'huile, on ajoute, à température ambiante, 20 cm³ de diméthylsulfoxyde anhydre.

On agite 30 minutes à 50 °C puis ramène à 25 °C et introduit 6 g de 7-diméthylamino 6,7,8,9-tétrahydro 5H-benzocyclohepten- 5-ol (cis), obtenus comme indiqué à l'exemple 1.

On agite 15 minutes puis ajoute 5,35 cm³ de p-bromotrifluoro-méthylbenzène. On porte à 50 °C pendant 4 heures, ramène à 20–25 °C, introduit 200 cm³ d'eau, extrait au chlorure de méthylène, lave à l'eau, sèche et amène à sec.

On obtient 12,35 g de produit brut que l'on purifie par chromatographie sur gel de silice. On élue par le mélange chloroforme-méthanol (7-3) et obtient après évaporation de l'éluant 7,3 g d'une huile orange correspondant à la N,N-diméthyl 6,7,8,9-tétrahydro 5-[[4-(trifluorométhyl)phényl]-oxy] 5H-benzocyclohepten-7-amine (cis).

Préparation du chlorhydrate

On dissout les 7,3 g d'amine obtenue ci-dessus dans 30 cm³ d'acétate d'éthyle, ajoute un excès d'acétate d'éthyle chlorhydrique, laisse cristalliser, essore, lave à l'acétate d'éthyle et sèche à 60 °C sous vide.

On obtient 7,2 g de chlorhydrate de N,N-diméthyl 6,7,8,9-tétrahydro 5-[[4-(trifluorométhyl)phényl]oxy] 5H-benzocyclohepten-7-amine (cis) fondant à ≃ 230 °C.

Analyse:

$C_{20}H_{22}F_3NO, HCl = 385,86.$

| | C % | H % | Cl % | F % | N % |
|---|---|---|---|---|---|
| Calculé: | 62,26 | 6,01 | 9,19 | 14,77 | 3,63 |
| Trouvé: | 62,4 | 5,9 | 9,2 | 14,5 | 3,4 |

Exemple 4

Chlorhydrate de N,N-diméthyl 5-[(4-nitrophényl)oxy] 6,7,8,9-tétrahydro 5H-benzocyclohepten-7-amine (trans)

A 1,44 g d'hydrure de sodium à 50% dans l'huile, on ajoute, à température ambiante 14,4 cm³ de diméthylsulfoxyde anhydre.

On porte une heure dans un bain à 55 °C, refroidit vers 20 °C, et ajoute 5,13 g de 7-diméthylamino 6,7,8,9-tétrahydro 5H-benzocyclohepten-5-ol (isomère trans obtenu comme indiqué à l'exemple 1).

On agite encore dix minutes à température ambiante puis ajoute en une seule fois 4,73 g de p-chloronitrobenzène.

On agite trois heures à température ambiante, reprend par du chlorure de méthylène, lave à l'eau, sèche sur sulfate de magnésium, filtre et distille à sec sous vide. On obtient 9,5 g de produit brut que l'on reprend par une solution d'acide chlorhydrique 0,5 N, élimine la fraction neutre par deux fois 100 cm³ d'éther, réunit les phases aqueuses, alcalinise par un fort excès d'ammoniaque, extrait par du chlorure de méthylène, sèche sur sulfate de magnésium, filtre et distille à sec sous vide. On obtient 8,5 g de N,N-diméthyl 5-[(4-nitrophényl)oxy] 6,7,8,9-tétrahydro 5H-benzocyclohepten-7-amine (trans).

Préparation du chlorhydrate

On dissout les 8,5 g obtenus ci-dessus dans 20 cm³ d'acétate d'éthyle, ajoute un fort excès d'acétate d'éthyle/acide chlorhydrique gazeux et amorce la cristallisation.

On laisse 12 heures en refrigérateur, essore, lave par de la méthyléthylcétone et sèche à 100 °C sous vide.

On obtient 5,5 g de chlorhydrate de N,N-diméthyl 5-[(4-nitrophényl)oxy] 6,7,8,9-tétrahydro

5H-benzocyclohepten-7-amine (trans) sous forme de cristaux incolores fondant à 192–196 °C.

Analyse:
$C_{19}H_{22}N_2O_3$, HCl = 362,85

| | C % | H % | N % | Cl % |
|---|---|---|---|---|
| Calculé: | 62,89 | 6,39 | 7,72 | 9,77 |
| Trouvé: | 63,0 | 6,5 | 7,8 | 10,1 |

Exemple 5

Chlorhydrate de N,N-diméthyl 6,7,8,9-tétrahydro 5-[[2-(trifluorométhyl)phényl]oxy] 5H-benzocyclohepten-7-amine (cis)

A 1,4 g d'hydrure de sodium à 50% dans l'huile, on ajoute 14 $cm^3$ de diméthylsulfoxyde, à température ambiante, chauffe 50 minutes à 50 °C, refroidit vers 20 °C et ajoute 4 g de 7-diméthylamino 6,7,8,9-tétrahydro 5H-benzocyclohepten-5-ol (isomère cis) obtenus comme indiqué à l'exemple 1.

On laisse 15 minutes à 20 °C puis ajoute 3,2 $cm^3$ d'o-bromo-trifluorométhyl benzène.

On porte à 100 °C ± 5 °C pendant deux heures 45 puis, ramène à 20 °C, reprend par du chlorure de méthylène, lave à l'eau, sèche puis amène à sec. On obtient 7,8 g de produit brut que l'on purifie par chromatographie sur gel de silice. On élue par du chlorure de méthylène renfermant 5% de méthanol. Après évaporation de l'éluant, on obtient 5,5 g de N,N-diméthyl 6,7,8,9-tétrahydro 5-[[2-(trifluorométhyl)phényl]oxy] 5H-benzocyclohepten-7-amine (cis) sous forme d'une huile.

Préparation du chlorhydrate

On dissout les 5,5 g obtenus ci-dessus, dans 30 $cm^3$ d'acétate d'éthyle, ajoute un excès d'acétate d'éthyle chlorhydrique, essore, lave à l'acétate d'éthyle puis sèche sous vide. On obtient 4,6 g de chlorhydrate de N,N-diméthyl 6,7,8,9-tétrahydro 5-[[2-(trifluorométhyl)phényl]oxy] 5H-benzocyclohepten-7-amine (cis) sous forme de cristaux incolores fondant à ≃220 °C.

Analyse:
$C_{20}H_{22}F_3NO$, HCl = 385,86

| | C % | H % | Cl % | F % | N % |
|---|---|---|---|---|---|
| Calculé: | 62,26 | 6,01 | 9,19 | 14,77 | 3,63 |
| Trouvé: | 62,1 | 6,1 | 9,4 | 14,5 | 3,5 |

Exemple 6

Chlorhydrate de N-méthyl 6,7,8,9-tétrahydro 5-[[4-(trifluorométhyl)phényl]oxy] 5H-benzocyclohepten-7-amine (cis)

Stade A

N-méthyl N-[6,7,8,9-tétrahydro 5-[[4-(trifluorométhyl)phényl]oxy] 5H-benzocyclohepten-7-yl] carbamate d'éthyle (cis)

a) Libération de la base

On empâte 7,4 g de chlorhydrate de N,N-diméthyl 6,7,8,9-tétrahydro 5-[[4-(trifluorométhyl)phényl]oxy] 5H-benzocyclohepten-7-amine (cis) obtenus comme indiqué à l'exemple 3, avec 70 $cm^3$ d'eau, ajoute un excès d'ammoniaque concentrée, extrait par du chlorure de méthylène, lave à l'eau, sèche puis amène à sec. On obtient 6,85 g de base correspondante.

b) Préparation du carbamate d'éthyle

On dissout les 6,85 g de base obtenus ci-dessus dans 60 $cm^3$ de benzène, ajoute sous azote 60 $cm^3$ de chloroformiate d'éthyle et porte au reflux pendant seize heures.

On amène à sec, reprend à l'éther, lave par de l'acide chlorhydrique N puis à l'eau, sèche sur sulfate de magnésium, traite par du charbon actif, filtre et amène à sec.

On obtient 8,17 g de produit brut que l'on purifie par chromatographie sur silice. On élue par du chlorure de méthylène et obtient après évaporation de l'éluant 7,1 g de produit attendu sous forme d'une huile incolore.

Stade B

Chlorhydrate de N-méthyl 6,7,8,9-tétrahydro 5-[[4-(trifluorométhyl)phényl]oxy] 5H-benzocyclohepten-7-amine (cis)

On dissout 7,1 g de N-méthyl N-[6,7,8,9-tétrahydro 5-[[4-(trifluorométhyl)phényl]oxy] 5H-benzocyclohepten-7-yl] carbamate d'éthyle obtenus ci-dessus dans 70 $cm^3$ de n-butanol, ajoute sous agitation et sous azote 7 g de potasse en pastilles, puis porte au reflux pendant 17 heures.

On amène à sec, reprend le résidu par du chlorure de méthylène, lave à l'eau, sèche puis amène à sec. On obtient 5,5 g de N-méthyl 6,7,8,9-tétrahydro 5-[[4-(trifluorométhyl)phényl]oxy] 5H-benzocyclohepten-7-amine (cis) sous forme d'une huile orange.

Préparation du chlorhydrate

On dissout les 5,5 g obtenus ci-dessus dans 30 $cm^3$ d'acétate d'éthyle, ajoute un excès d'acétate d'éthyle chlorhydrique, laisse cristalliser, essore, lave à l'acétate d'éthyle et sèche.

On obtient 5,4 g de chlorhydrate attendu sous forme de cristaux blancs fondant à ≃220 °C.

Analyse:
$C_{19}H_{20}F_3NO$, HCl = 371,84

| | C % | H % | Cl % | F % | N % |
|---|---|---|---|---|---|
| Calculé: | 61,37 | 5,69 | 9,53 | 15,33 | 3,77 |
| Trouvé: | 61,3 | 5,8 | 9,5 | 15,2 | 3,6 |

Exemple 7

Chlorhydrate de 5-[(4-bromophényl)oxy] N,N-diméthyl 6,7,8,9-tétrahydro 5H-benzocyclohepten-7-amine (cis)

A 0,820 g d'hydrure de sodium à 50% dans l'huile, on ajoute 8 $cm^3$ de diméthyl sulfoxyde, chauffe 45 minutes à 50 °C, refroidit vers 20 °C et ajoute 3 g de 7-diméthylamino 6,7,8,9-térahydro 5H-benzocyclohepten-5-ol (cis) obtenus comme indiqué à l'exemple 1.

On laisse 15 minutes à température ambiante puis ajoute 2 $cm^3$ de p-bromofluorobenzène. On porte à 90 °C ±5 °C pendant une heure, ramène à

20 °C, reprend par du chlorure de méthylène, lave à l'eau, sèche puis amène à sec. On obtient 6 g de 5-[(4-bromophényl)oxy] N,N-diméthyl 6,7,8,9-tétrahydro 5H-benzocyclohepten-7-amine (cis) sous forme d'une huile jaune.

Préparation du chlorhydrate

On dissout les 6 g obtenus ci-dessus, dans 30 cm³ d'acétate d'éthyle, ajoute un excès d'acétate d'éthyle chlorhydrique, laisse cristalliser pendant une heure à 20 °C, essore, lave à l'acétate d'éthyle puis sèche. On obtient 4,8 g de chlorhydrate de 5-[(4-bromophényl)oxy] N,N-diméthyl 6,7,8,9-tétrahydro 5H-benzocyclohepten-7-amine (cis) sous forme de cristaux incolores fondant à environ 230 °C.

Analyse:

$C_{19}H_{22}BrNO$, HCl = 396,76

| | C % | H % | Br % | Cl % | N % |
|---|---|---|---|---|---|
| Calculé: | 57,52 | 5,84 | 20,14 | 8,94 | 3,53 |
| Trouvé: | 57,2 | 6,0 | 19,7 | 8,8 | 3,6 |

Exemple 8

Chlorhydrate de N,N-diméthyl 5-[(2-nitrophényl)oxy] 6,7,8,9-tétrahydro 5H-benzocyclohepten-7-amine (cis)

A 1,1 g d'hydrure de sodium à 50% dans l'huile, on ajoute 11 cm³ de diméthylsulfoxyde, porte 30 minutes dans un bain à 70 °C, refroidit vers 20 °C et ajoute 4,1 g de 7-diméthylamino 6,7,8,9-tétrahydro 5H-benzocyclohepten-5-ol (cis) obtenus comme indiqué à l'exemple 1.

On agite 10 minutes à température ambiante puis ajoute 6,32 g d'o-chloronitrobenzène en refroidissant par un bain de glace et agite 30 minutes vers 20 °C, reprend par du chlorure de méthylène, lave à l'eau, sèche sur sulfate de magnésium, filtre et distille à sec sous vide. On obtient 12,8 g de N,N-diméthyl 5-[(2-nitrophényl)oxy] 6,7,8,9-tétrahydro 5H-benzocyclohepten-7-amine (cis) sous forme d'une huile brune.

Préparation du chlorhydrate

On reprend les 12,8 g obtenus ci-dessus par 30 cm³ d'eau, ajoute 2 cm³ d'acide chlorhydrique 2N, essore, lave à l'eau, à l'éther et sèche à 65 °C sous vide. On recueille 5,9 g de chlorhydrate de N,N-diméthyl 5-[(2-nitrophényl)oxy] 6,7,8,9-tétrahydro 5H-benzocyclohepten-7-amine (cis) que l'on fait recristalliser dans l'éthanol au reflux. On obtient un produit se présentant sous la forme de paillettes jaunes fondant à ≃240 °C.

Analyse:

$C_{19}H_{22}N_2O_3$,HCl = 362,84

| | C % | H % | N % | Cl % |
|---|---|---|---|---|
| Calculé: | 62,89 | 6,39 | 7,72 | 9,77 |
| Trouvé: | 62,8 | 6,5 | 7,6 | 9,8 |

Exemple 9

Chlorhydrate de N-méthyl 5-[(4-nitrophényl)-oxy] 6,7,8,9-tétrahydro 5H-benzocyclohepten-7-amine (cis)

A 652 mg de N,N-diméthyl 5-[(4-nitrophényl)oxy] 6,7,8,9-tétrahydro 5H-benzocyclohepten-7-amine (cis) (obtenus comme indiqué à l'exemple 1), dans 10 cm³ d'acétone, on ajoute 348 mg d'azocarboxylate de diéthyle. On porte au reflux 3 heures 30, distille l'acétone sous vide, ajoute 5 cm³ d'acide chlorhydrique N et agite 26 heures à température ambiante.

On alcalinise par de l'ammoniaque, extrait au chlorure de méthylène, lave à l'eau, sèche sur sulfate de magnésium, filtre et distille à sec sous vide.

On obtient 1,1 g de produit brut que l'on purifie par chromatographie sur silice dans le mélange chloroforme-méthanol (7-3).

On récupère 400 mg de N-méthyl 5-[(4-nitrophényl)oxy] 6,7,8,9-tétrahydro 5H-benzocyclohepten-7-amine (cis).

Préparation du chlorhydrate

On dissout le produit obtenu ci-dessus dans l'acétate d'éthyle chlorhydrique et recueille finalement 380 mg de chlorhydrate de N-méthyl 5-[(4-nitrophényl)oxy] 6,7,8,9-tétrahydro 5H-benzocyclohepten-7-amine (cis) fondant à 265 °C.

Analyse:

$C_{18}H_{20}N_2O_3$, HCl = 348,83

| | C % | H % | Cl % | N % |
|---|---|---|---|---|
| Calculé: | 61,97 | 6,07 | 10,16 | 8,03 |
| Trouvé: | 61,9 | 6,2 | 10,2 | 7,8 |

Exemple 10

Chlorhydrate de N,N-diméthyl 5-[(2-méthoxyphényl)oxy] 6,7,8,9-tétrahydro 5H-benzocyclohepten-7-amine (cis)

On ajoute goutte à goutte 14 cm³ de diméthyl sulfoxyde à 1,4 g d'hydrure de sodium en suspension dans l'huile à 50%, porte à 60 °C pendant 25 minutes, ramène à 25 °C puis ajoute 5 g de 7-diméthylamino 6,7,8,9-tétrahydro 5H-benzocyclohepten-5-ol (cis) obtenu comme indiqué à l'exemple 1.

On laisse 15 minutes à température ambiante puis ajoute 10,7 cm³ d'orthofluoroanisole.

On porte à 90° ± 5 °C pendant 4 heures 30 puis ramène à 25 °C, reprend par du chlorure de méthylène, lave à l'eau, sèche puis amène à sec sous vide à 60 °C. On obtient 8,5 g de produit brut que l'on purifie par chromatographie sur gel de silice. On élue par le mélange chloroforme-méthanol (7-3) et obtient, après évaporation de l'éluant 5,4 g de N,N-diméthyl 5-[(2-méthoxyphényl)oxy] 6,7,8,9-tétrahydro 5H-benzocyclohepten-7-amine (cis) sous forme d'une huile orange.

Préparation du chlorhydrate

On dissout les 5,4 g obtenus ci-dessus dans 30 cm³ d'acétate d'éthyle, ajoute un excès d'acétate d'éthyle chlorhydrique, concentre, essore, lave à l'acétate d'éthyle puis sèche sous vide à 60 °C. Après recristallisation dans l'éthanol, on obtient 4,45 g de chlorhydrate de N,N-diméthyl 5-[(2-méthoxyphényl)oxy] 6,7,8,9-tétrahydro 5H-benzocyclohepten-7-amine (cis) sous forme de cristaux incolores fondant à 212 °C puis 222 °C.

Analyse:

$C_{20}H_{25}NO_2$, HCl = 347,89

| | C % | H % | Cl % | N % |
|---|---|---|---|---|
| Calculé: | 69,05 | 7,53 | 10,19 | 4,03 |
| Trouvé: | 68,7 | 7,5 | 10,1 | 3,8 |

Exemple 11

Chlorhydrate de N,N-diméthyl 5-[(4-méthoxyphényl)oxy] 6,7,8,9-tétrahydro 5H-benzocyclohepten-7-amine (cis)

On ajoute goutte à goutte 12 $cm^3$ de diméthyl sulfoxyde à 1,16 g d'hydrure de sodium en suspension à 50% dans l'huile et porte à 60 °C pendant 35 minutes.

On ramène à 25 °C puis ajoute 4,1 g de 7-diméthylamino 6,7,8,9-tétrahydro 5H-benzocyclohepten-5-ol (cis) obtenus comme indiqué à l'exemple 1 et 15 minutes plus tard 8,8 $cm^3$ de p-fluoroanisole.

On chauffe 46 heures à 90 °C, ramène à 25 °C, reprend par 150 $cm^3$ de chlorure de méthylène, lave à l'eau, sèche et amène à sec sous vide. On obtient 5,7 g de produit brut que l'on purifie par chromatographie sur gel de silice. On élue par le mélange chloroforme-méthanol (7-3) et obtient après évaporation de l'éluant 1,7 g de N,N-diméthyl 5-[(4-méthoxyphényl)oxy] 6,7,8,9-tétrahydro 5H-benzocyclohepten-7-amine (cis) sous forme d'une huile orange.

Préparation du chlorhydrate

On dissout les 1,7 g obtenu ci-dessus dans 15 $cm^3$ d'acétate d'éthyle, ajoute un excès d'acétate d'éthyle chlorhydrique, concentre, essore, lave à l'acétate d'éthyle et sèche sous vide. On obtient, après recristallisation dans l'éthanol 1,60 g de chlorhydrate de N,N-diméthyl 5-[(4-méthoxyphényl)oxy] 6,7,8,9-tétrahydro 5H-benzocyclohepten-7-amine (cis) sous forme de cristaux incolores fondant à 215 °C.

Analyse:

$C_{20}H_{25}NO_2$, HCl =347,89

| | C % | H % | M Cl % | N % |
|---|---|---|---|---|
| Calculé: | 69,05 | 7,53 | 10,19 | 4,03 |
| Trouvé: | 68,9 | 7,5 | 10,1 | 3,9 |

Exemple 12

Chlorhydrate de 5-[(4-chlorophényl)oxy] N,N-diméthyl 6,7,8,9-tétrahydro 5H-benzocyclohepten-7-amine (cis)

A 60 mg d'hydrure de sodium en suspension à 50% dans l'huile, on ajoute, à température ambiante, 0,6 $cm^3$ de diméthylsulfoxyde.

On porte 20 minutes à 50 °C, ramène à 20 °C puis introduit 205 mg de 7-diméthylamino 6,7,8,9-tétrahydro 5H-benzocyclohepten-5-ol (cis) obtenus comme indiqué à l'exemple 1.

On laisse 15 minutes sous agitation puis introduit 0,13 $cm^3$ de p-chlorofluorobenzène. On laisse une heure à 20 °C, chauffe une heure 30 à 50 °C puis trois heures à 100 °C. On ramène à 20 °C, reprend par 30 $cm^3$ de chlorure de méthylène, lave à l'eau, sèche et amène à sec. On obtient 300 mg de produit brut que l'onpurifie par chromatographie sur silice. On élue par le mélange chloroforme-méthanol (7-3). Après évaporation de l'éluant, on recueille 250 mg de 5-[(4-chlorphényl)oxy] N,N-diméthyl 6,7,8,9-tétrahydro 5H-benzocyclohepten-7-amine (cis) sous forme d'une huile.

Préparation du chlorhydrate

On dissout les 250 mg obtenus ci-dessus dans 2 $cm^3$ d'acétate d'éthyle, ajoute un excès d'acétate d'éthyle chlorhydrique, concentre, essore, lave à l'acétate d'éthyle et sèche. On obtient 240 mg de chlorhydrate de 5-[(4-chlorophényl)oxy] N,N-diméthyl 6,7,8,9-tétrahydro 5H-benzocyclohepten-7-amine (cis) sous forme de cristaux incolores fondant à 215 °C.

Analyse:

$C_{19}H_{22}ClNO$, HCl = 352,31

| | C % | H % | Cl % | N % |
|---|---|---|---|---|
| Calculé: | 64,77 | 6,58 | 20,13 | 3,98 |
| Trouvé: | 64,7 | 6,8 | 19,9 | 3,9 |

Exemple 13

Chlorhydrate de N,N-diméthyl 5-(phénoxy) 6,7,8,9-tétrahydro 5H-benzocyclohepten-7-amine (cis)

Stade A

Préparation de la base

a) Libération de la base

On empâte 330 mg de chlorhydrate de 5-[(4-bromophényl)oxy] N,N-diméthyl 6,7,8,9-tétrahydro 5H-benzocyclohepten-7-amine (cis) préparés comme indiqué à l'exemple 7 avec 15 $cm^3$ d'eau, ajoute 5 $cm^3$ d'ammoniaque (d = 0.90), extrait à l'acétate d'éthyle, lave à l'eau, sèche puis amène à sec. On obtient 310 mg de base correspondante.

b) Hydrogénolyse de la base

On dissout les 310 mg obtenus ci-dessus dans 9 $cm^3$ d'éthanol, ajoute 0,35 $cm^3$ de triéthylamine séchée sur potasse puis 35 mg de palladium à 10% sur charbon. On hydrogène à 20 °C, sous agitation et pression ambiante (30 $cm^3$ d'hydrogène). On filtre, amène à sec, ajoute de la lessive de soude, extrait à l'acétate d'éthyle, lave à l'eau, sèche puis amène à sec. On obtient 235 mg de N,N-diméthyl 5-(phénoxy) 6,7,8,9-tétrahydro 5H-benzocyclohepten-7-amine (cis) sous forme d'une huile jaune.

Stade B

Préparation du chlorhydrate

On dissout les 235 mg obtenus ci-dessus dans 3 $cm^3$ d'acétate d'éthyle, ajoute un excès d'acétate d'éthyle chlorhydrique, concentre, essore, lave à l'acétate d'éthyle puis sèche.

On obtient 215 mg de chlorhydrate de N,N-diméthyl 5-(phénoxy) 6,7,8,9-tétrahydro 5H-benzocyclohepten-7-amine (cis) sous forme de cristaux incolores fondant à 225 °C.

Analyse:
$C_{19}H_{23}NO$, HCl = 317,86

| | C % | H % | Cl % | N % |
|---|---|---|---|---|
| Calculé: | 71,80 | 7,61 | 11,15 | 4,41 |
| Trouvé: | 71,6 | 7,7 | 11,0 | 4,3 |

Exemple 14

Chlorhydrate de N,N-diméthyl 5-[(4-fluorophényl)oxy] 6,7,8,9-tétrahydro 5H-benzocyclohepten-7-amine (cis)

On ajoute 0,6 $cm^3$ de diméthylsulfoxyde à 60 mg d'hydrure de sodium en suspension à 50% dans l'huile. On porte 25 minutes à 60 °C, ramène à 25 °C puis introduit 205 mg de 7-diméthylamino 6,7,8,9-tétrahydro [5H]-benzocyclohepten-5-ol-(cis) obtenus comme indiqué à l'exemple 1 et 15 minutes plus tard, 0,4 $cm^3$ de p-difluorobenzène.

On chauffe pendant 6 heures 45 à 90 °C puis ramène à 20 °C, reprend par du chlorure de méthylène, lave à l'eau, sèche et amène à sec. On dissout les 350 mg de produit brut obtenus, dans 10 $cm^3$ d'acide chlorhydrique N, lave à l'éther, alcalinise la fraction aminée par de la lessive de soude, extrait par du chlorure de méthylène, lave à l'eau, sèche puis amène à sec. On obtient 270 mg de N,N-diméthyl 5-[(4-fluorophényl)oxy] 6,7,8,9-tétrahydro 5H-benzocyclohepten-7-amine (cis) sous forme d'une huile.

Préparation du chlorhydrate

On dissout les 270 mg obtenus ci-dessus dans 3 $cm^3$ d'acétate d'éthyle, ajoute un excès d'acétate d'éthyle chlorhydrique, concentre, essore, lave à l'acétate d'éthyle puis sèche.

On obtient 220 mg de chlorhydrate de N,N-diméthyl 5-[(4-fluorophényl)oxy] 6,7,8,9-tétrahydro 5H-benzocyclohepten-7-amine (cis) sous forme de cristaux incolores fondant à 208 °C.

Analyse:
$C_{19}H_{22}FNO$, HCl = 335,85

| | C % | H % | Cl % | F % | N % |
|---|---|---|---|---|---|
| Calculé: | 67,95 | 6,90 | 10,56 | 5,66 | 4,17 |
| Trouvé: | 67,6 | 7,1 | 10,7 | 5,3 | 4,1 |

Exemple 15

Chlorhydrate de 5-[(2-chlorophényl)oxy] N,N-diméthyl 6,7,8,9-tétrahydro 5H-benzocyclohepten-7-amine (cis)

A 65 mg d'hydrure de sodium en suspension dans l'huile à 50% on ajoute 0,6 $cm^9$ de diméthylsulfoxyde, porte 30 minutes à 25 °C puis à 60 °C. On ramène à 25 °C puis ajoute 205 mg de 7-diméthylamino 6,7,8,9-tétrahydro 5H-benzocyclohepten-5-ol (cis) obtenus comme indiqué à l'exemple 1.

On laisse 15 minutes à température ambiante puis ajoute 0,15 $cm^3$ d'o-chlorofluorobenzène. On porte à 80 °C pendant 30 minutes puis ramène à 20 °C, reprend par du chlorure de méthylène, lave à l'eau, sèche et amène à sec.

On obtient 370 mg de 5-[(2-chlorophényl)oxy] N,N-diméthyl 6,7,8,9-tétrahydro 5H-benzocyclohepten-7-amine (cis) sous forme d'une huile orange.

Préparation du chlorhydrate

On dissout les 370 mg obtenus ci-dessus dans 4 $cm^3$ d'acétate d'éthyle, ajoute un excès d'acétate d'éthyle chlorhydrique, concentre, lave à l'acétate d'éthyle chlorhydrique puis sèche.

On obtient 315 mg de chlorhydrate de 5-[(2-chlorophényl)oxy] N,N-diméthyl 6,7,8,9-tétrahydro 5H-benzocyclohepten-7-amine (cis) sous forme de cristaux incolores fondant à ≃240 °C.

Analyse:
$C_{19}H_{22}ClNO$, HCl = 352,31

| | C % | H % | Cl % | N % |
|---|---|---|---|---|
| Calculé: | 64,77 | 6,58 | 20,12 | 3,98 |
| Trouvé: | 64,8 | 6,7 | 20,2 | 3,9 |

Exemple 16

Chlorhydrate de N,N-diméthyl 5-[(3-nitrophényl)oxy] 6,7,8,9-tétrahydro 5H-benzocyclohepten-7-amine (cis)

A 1,4 g d'hydrure de sodium en suspension dans l'huile à 50%, on ajoute 14 $cm^3$ de diméthylsulfoxyde, porte en 35 minutes à 25 °C puis à 60 °C. On ramène à 25 °C puis ajoute 5 g 7-diméthylamino 6,7,8,9-tétrahydro 5H-benzocyclohepten-5-ol (cis) préparés comme indiqué à l'exemple 1.

On laisse 15 minutes à température ambiante puis ajoute 5,3 $cm^3$ de m-fluoronitrobenzène. On ramène la température à 25 °C puis garde ainsi 30 minutes.

On extrait par du chlorure de méthylène, lave à l'eau salée, sèche puis amène à sec.

On obtient 10,8 g de N,N-diméthyl 5-[(3-nitrophényl)oxy] 6,7,8,9-tétrahydro 5H-benzocyclohepten-7-amine (cis) sous forme d'une gomme rouge.

Préparation du chlorhydrate

On reprend les 10,8 g obtenus ci-dessus par 17 $cm^3$ d'acide chlorhydrique 2N, ajoute de l'éther, glace, essore, lave à l'eau puis à l'éther.

On sèche à 80 °C sous pression réduite et obtient 4 g de chlorhydrate attendu que l'on redissout dans le méthanol bouillant. On ajoute de l'acétate d'éthyle, filtre, concentre, laisse 12 heures à 20 °C, essore, lave à l'acétate d'éthyle et sèche sous vide.

On obtient 3,5 g de chlorhydrate de N,N-diméthyl 5-[(3-nitrophényl)oxy] 6,7,8,9-tétrahydro 5H-benzocyclohepten-7-amine (cis) sous forme d'un produit de couleur beige fondant à 216 °C.

Analyse:
$C_{19}H_{22}N_2C_3$, HCl = 362,85

| | C % | H % | Cl % | N % |
|---|---|---|---|---|
| Calculé: | 62,89 | 6,39 | 9,77 | 7,72 |
| Trouvé: | 62,6 | 6,3 | 9,9 | 7,4 |

Exemple 17

N,N-diméthyl 5-[(4-nitro 3-(trifluorométhyl)-phényl]oxy] 6,7,8,9-tétrahydro 5H-benzocyclohepten 7-amine (cis)

A 1,5 g d'hydrure de sodium, en dispersion à 50% dans l'huile, on ajoute 30 $cm^3$ de diméthylsulfoxyde anhydre. On agite 20 minutes à 75°C, puis ramène à 20°C et introduit 5,1 g de 7-diméthylamino 6,7,8,9-tétrahydro 5H-benzocyclohepten 5-ol (cis) (composé décrit à l'exemple 1).

On agite 10 minutes, refroidit au bain de glace et ajoute 6,7 g de 5-chloro-2-nitro trifluorométhyl benzène. On agite 30 minutes à 70°C, ramène à 20°C, extrait au chlorure de méthylène, lave à l'eau salée, sèche et amène à sec.

On obtient 13 g de produit brut que l'on reprend par une solution d'acide chlorhydrique, élimine la fraction neutre à l'éther, alcalinise par un excès d'ammoniaque, extrait au chlorure de méthylène, sèche, distille à sec et recueille 9,9 g d'huile brune.

On reprend l'huile obtenue par 200 $cm^3$ d'éther isopropylique à 50°C, traite au charbon actif, filtre, distille à sec sous vide, ajoute 10 $cm^3$ d'éther isopropylique et laisse cristalliser. On essore les cristaux et obtient 5,95 g de produit qui, recristallisé dans l'éther de pétrole, puis l'éther isopropylique, donne 3,8 g de cristaux beiges fondant à 75°C.

Analyse:
$C_{20}H_{21}F_3N_2O_3$ = 394,39

| | C % | H % | F % | N % |
|---|---|---|---|---|
| Calculé: | 60,90 | 5,37 | 14,45 | 7,10 |
| Trouvé: | 60,6 | 5,4 | 14,2 | 7,1 |

Exemple 18

Chlorhydrate de 3-chloro N,N-diméthyl 5-[(4-nitro-phényl)oxy] 6,7,8,9-tétrahydro 5H-benzocyclohepten 7-amine (trans)

A 1,2 g d'hydrure de sodium en dispersion à 50% dans l'huile, on ajoute sous azote 12 $cm^3$ de diméthylsulfoxyde anhydre. On agite 30 minutes à 60°C, refroidit à 25°C et ajoute 4,8 g de 3-chloro 7-diméthylamino 6,7,8,9-tétrahydro 5H-benzocyclohepten-5-ol (trans).

On agite encore 15 minutes vers 25°C et ajoute 6,3 g de p-chloronitro benzène, puis agite encore une heure. On ajoute 500 $cm^3$ d'eau, extrait au chlorure de méthylène, sèche sur sulfate de magnésium, filtre et distille à sec sous vide.

Préparation du chlorhydrate:

On reprend par 130 $cm^3$ d'acide chlorhydrique N et laisse reposer 20 minutes, essore, lave à l'éther, à l'eau, essore et sèche sous vide.

On obtient 7,3 g de produit qui, recristallisé dans le méthanol et l'acétate d'éthyle, donne 5 g de cristaux incolores fondant à 230°C.

Analyse:
$C_{19}H_{21}Cl\,N_2O_3$, HCl = 397,30

| | C % | H % | N % | Cl % |
|---|---|---|---|---|
| Calculé: | 57,44 | 5,58 | 7,05 | 17,85 |
| Trouvé: | 57,4 | 5,7 | 7,1 | 17,8 |

Le 3-chloro 7-diméthylamino 6,7,8,9-tétrahydro 5H-benzocyclohepten 5-ol (trans) de départ peut être obtenu de la façon suivante:

Stade A

On agite 1 heure à 20°C, 9,6 g de 3-chloro 8,9-dihydro 5H-benzocyclohepten 5-one (préparée selon la demande de brevet français 2319332) avec 15 $cm^3$ de diméthylamine en solution éthanolique à 33%, amène à sec et obtient 11,6 g d'huile orange.

On dissout les 11,6 g de produit brut dans 60 $cm^3$ d'acétate d'éthyle, ajoute sous atmosphère inerte à 5°C un excès d'acétate d'éthyle chlorhydrique, laisse 2 heures à 5°C, essore, lave à l'acétate d'éthyle, sèche et obtient 9,6 g de chlorhydrate de 3-chloro 7-diméthylamino 6,7,8,9-tétrahydro 5H-benzocyclohepten 5-one sous forme de solide blanc fondant à 146°C servant tel quel au stade suivant.

Stade B

On agite 10 minutes à 10°C, 1 g de chlorhydrate de 3-chloro 7-diméthylamino 6,7,8,9-tétrahydro 5H-benzocyclohepten 5-one sous atmosphère inerte avec 10 $cm^3$ de méthanol, 1 $cm^3$ d'eau et 1 g de borohydrure de sodium, puis verse dans 50 $cm^3$ d'eau, extrait au chlorure de méthylène, sèche et distille à sec sous vide.

On obtient 900 mg d'huile jaune que l'on chromatographie sur silice, élue par un mélange acétate d'éthyle–triéthylamine 8/2, récupère une fraction homogène de 250 mg d'huile jaune au Rf 0,12 que l'on cristallise dans l'éther et recristallise dans l'éther isopropylique, pour obtenir 100 mg de cristaux incolores de produit attendu fondant à 110°C.

Analyse:
$C_{19}H_{18}ClNO$ = 239,75

| | C % | H % | N % | Cl % |
|---|---|---|---|---|
| Calculé: | 65,13 | 7,57 | 5,84 | 14,79 |
| Trouvé: | 65,3 | 7,6 | 5,7 | 14,9 |

Exemple 19

Chlorhydrate de 3-chloro N,N-diméthyl 5-[(4-nitrophényl)oxy] 6,7,8,9-tétrahydro 5H-benzocyclohepten 7-amine (cis)

Selon un procédé identique à celui utilisé pour l'exemple 18, à partir de 4,8 g de 3-chloro 7-diméthylamino 6,7,8,9-tétrahydro 5H-benzocyclohepten 5-ol (cis), on obtient 5,3 g de cristaux incolores fondant à 255°C.

Analyse:
$C_{19}H_{21}ClN_2O_3$, HCl = 397,30

| | C % | H % | N % | Cl % |
|---|---|---|---|---|
| Calculé: | 57,44 | 5,58 | 7,05 | 17,85 |
| Trouvé: | 57,2 | 5,7 | 6,9 | 18,0 |

Le 3-chloro 7-diméthylamino 6,7,8,9-tétrahydro 5H-benzocyclohepten 5-ol (cis) de départ peut être obtenu selon la préparation décrite à l'exemple 18.

On récupère lors de la chromatographie des 900 mg de produit brut une fraction homogène de 360 mg d'huile jaune au Rf 0,06 qui, après purification dans l'éther, donnent 160 mg de cristaux incolores fondant à 140 °C.

Exemple 20

On a préparé des comprimés répondant à la formule:

- Fumarate de N,N-diméthyl 5-[(4-nitrophényl)oxy] 6,7,8,9-tétrahydro 5H-benzocyclohepten-7-amine (cis) 25 mg
- Excipient q.s. pour un comprimé terminé à 200 mg

(Détail de l'excipient: lactose, amidon, talc, stéarate de magnésium.)

Exemple 21

On a préparé des comprimés répondant à la formule:

- Chlorhydrate de N,N-diméthyl 5-[(4-nitrophényl)oxy] 6,7,8,9-tétrahydro 5H-benzocyclohepten-7-amine (trans) 25 mg
- Excipient q.s. pour un comprimé terminé à 200 mg

(Détail de l'excipient: lactose, amidon, talc, stéarate de magnésium.)

Etude pharmacologique

1) Etude biochimique

a) Inhibition de l'«uptake» (captation) de la sérotonine in vitro.

L'inhibition de l'uptake de la sérotonine (5 HT) est mesurée dans des synaptosomes non purifiés, préparés à partir de cerveau entier de rat (femme 19–21 jours) suivant la technique de Kannengiesser et coll. (Biochemical Pharmacology, 22, 73, 1973).

Les produits à diverses concentrations sont mis à incuber avec la préparation à 37 °C pendant 5 minutes en présence de la 14C-5 HT à une concentration de $10^{-7}$M.

La concentration inhibitrice 50 (C.I.50) d'un produit, c'est-à-dire la dose qui inhibe de 50% la captation de la 14C-5 HT dans les synaptosomes, est déterminée graphiquement.

Les résultats obtenus figurent dans le tableau ci-après:

| Produit décrit à l'exemple | Concentration inhibitrice 50 (C.I. 50) (M) |
|---|---|
| 1 | $3,4 \times 10^{-7}$ |
| 2 | $1,4 \times 10^{-5}$ |
| 3 | $7,6 \times 10^{-7}$ |
| 4 | $8,3 \times 10^{-8}$ |
| 5 | $2,3 \times 10^{-6}$ |
| 6 | $1,9 \times 10^{-6}$ |
| 7 | $3,2 \times 10^{-7}$ |
| 8 | $4 \times 10^{-6}$ |
| 10 | $1,3 \times 10^{-6}$ |
| 11 | $1,3 \times 10^{-6}$ |
| 17 | $9,5 \times 10^{-7}$ |
| 18 | $1,5 \times 10^{-6}$ |
| 19 | $7,1 \times 10^{-6}$ |

b) Inhibition de l'«uptake» (captation) de la sérotonine in vivo.

Les produits étudiés sont administrés par voie intrapéritonéale à des lots de rats femelles de 19 à 21 jours à une dose de 5 mg/kg.

Le cerveau des animaux est prélevé soit une heure, soit 24 heures après l'administration des produits.

Des synaptosomes sont préparés et mis à incuber en présence de la 14-C-5 HT comme indiqué précédemment.

Le pouvoir relatif des produits à inhiber la captation de la 14C-5 HT est estimé par rapport à un essai effectué sur des animaux ayant seulement reçu le solvant.

L'activité est exprimée en pourcentage d'inhibition de l'uptake pour chaque temps.

Les résultats obtenus figurent dans le tableau ci-après:

| Produit décrit à l'exemple | Pourcentage d'inhibition après une heure | vingt-quatre heures |
|---|---|---|
| 1 | 58% ± 4 | 67% ± 2 |
| 4 | 61% ± 2 | 39% ± 4 |
| 6 | 41% ± 3 | 61% ± 3 |
| 7 | 40% ± 3 | 65% ± 2 |

Les résultats obtenus montrent que les produits possèdent de très intéressantes propriétés sérotoninergiques étalées dans le temps.

2) Etude de l'activité anorexigène

L'activité anorexigène est évaluée chez le chien suivant la méthode de Adams et Greene (J. Pharm. Sci. 1964, 53, 1405).

Tous les animaux reçoivent comme alimentation, exclusivement de la pâtée U.A.R. sur la base de 80 g par jour et par animal. Le jour de l'essai d'une substance présumée anorexigène, la ration quotidienne individuelle des animaux est fragmentée en boulettes sensiblement égales (15 à 20 g) qui leur sont offertes à raison d'une toutes les 10 minutes pendant huit heures. Normalement les animaux acceptent régulièrement les boulettes successives qui leur sont présentées. Le refus traduit l'efficacité anorexiante du composé étudié, administré en gélules dans la première boulette.

Le nombre horaire de refus est déterminé sur l'ensemble des animaux traités à une même dose et exprimé en pourcentage du nombre total horaire de boulettes présentées, soit 6 par animal.

Au moment de l'essai, les animaux sont à jeun depuis 15 heures environ mais en ayant toujours eu de l'eau de boisson à leur disposition. Ils en sont par contre privés au cours de l'essai.

Les essais sont pratiqués sur un groupe de 7 chiens bâtards adultes mâles et femelles, d'un poids compris entre 11,4 et 14 kg (moyenne 12,1 kg) sélectionnés en fonction de leur réponse à une faible dose de fenfluramine (5 mg/kg).

Dans les conditions de l'essai, le produit de l'exemple 1 exerce une importante activité anorexigène par voie orale, à la dose de 10 mg/kg.

3) Etude de la toxicité aiguë:

Les doses létales 50 (DL 50) des différents produits ont été évaluées chez les souris après administration par voie intrapéritonéale.

La mortalité est constatée en 48 heures.

Les DL 50 approchées figurent dans le tableau ci-après:

| Produit décrit à l'exemple | DL 50 en mg/kg I.P. |
|---|---|
| 1 | 100 |
| 2 | 200 |
| 3 | 150 |
| 4 | 100 |
| 5 | 200 |
| 6 | 400 |
| 7 | 150 |
| 8 | 150 |
| 17 | >400 |
| 18 | 100 |
| 19 | >200 |

**Revendications**

1. Dérivés de la 5H-benzocyclohepten-7-amine, ainsi que leurs sels d'addition avec les acides minéraux ou organiques, caractérisés en ce qu'ils répondent à la formule générale:

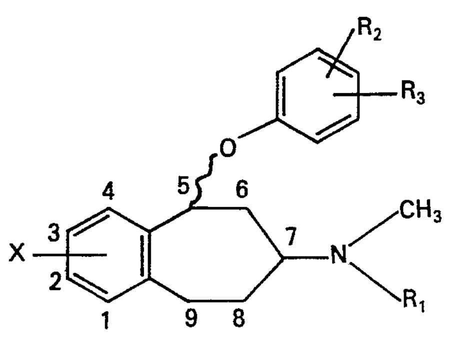

(I')

dans laquelle $R_1$ représente un atome d'hydrogène ou un radical méthyle, $R_2$ et $R_3$, identiques ou différents, représentent un atome d'hydrogène, de fluor, de chlore ou de brome, un radical nitro, amino, trifluorométhyle ou méthoxy et X représente un atome d'hydrogène ou d'halogène.

2. Dérivés selon la revendication 1, ainsi que leurs sels d'addition avec les acides minéraux ou organiques, caractérisés en ce qu'ils répondent à la formule générale:

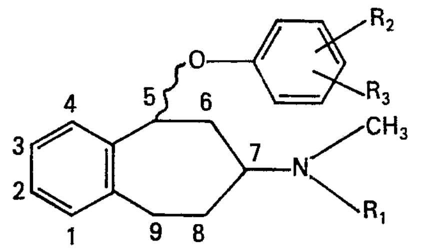

(I)

dans laquelle $R_1$, $R_2$ et $R_3$ sont définis comme à la revendication 1.

3. Dérivés répondant à la formule générale (I) de la revendication 2, ainsi que leurs sels d'addition avec les acides minéraux ou organiques, caractérisés en ce que dans ladite formule (I), $R_1$ représente un radical méthyle, $R_2$ et $R_3$ ont la signification indiquée à la revendication 1.

4. Dérivés répondant à la formule générale (I) de la revendication 2, ainsi que leurs sels d'addition avec les acides minéraux ou organiques, caractérisés en ce que dans ladite formule (I), $R_1$ représente un radical méthyle et l'un au moins de $R_2$ et $R_3$ représente un atome d'hydrogène.

5. L'un quelconque des dérivés répondant à la formule (I) dont les noms suivent:
- les isomères cis et trans de la N,N-diméthyl 5-[(4-nitrophényl)oxy] 6,7,8,9-tétrahydro 5H-benzocyclohepten-7-amine,
- l'isomère cis de la N,N-diméthyl 6,7,8,9-tétrahydro 5-[[4-(trifluorométhyl) phényl]oxy] 5H-benzocyclohepten-7-amine,
- l'isomère cis de la N,N-diméthyl 5-[(4-bromophényl)oxy] 6,7,8,9-tétrahydro 5H-benzocyclohepten-7-amine, ainsi que leurs sels d'addition avec les acides minéraux ou organiques.

6. Le fumarate de N,N-diméthyl 5-[(4-nitrophényl)oxy] 6,7,8,9-tétrahydro 5H-benzocyclohepten-7-amine (isomère cis).

7. Procédé de préparation des dérivés, ainsi que de leurs sels, tels que définis par la formule I' de la revendication 1, ledit procédé étant caractérisé en ce que l'on fait réagir un produit de formule:

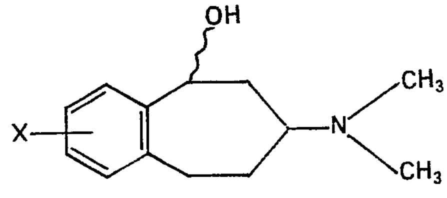

(II)

dans laquelle X représente un atome d'hydrogène ou d'halogène, avec un produit de formule:

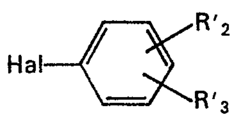

(III)

dans laquelle Hal représente un atome de fluor, de chlore ou de brome, $R'_2$ représente un atome de fluor, de chlore ou de brome, un radical nitro, trifluorométhyle ou méthoxy et $R'_3$ représente un

atome d'hydrogène ou $R'_2$, pour obtenir un produit de formule:

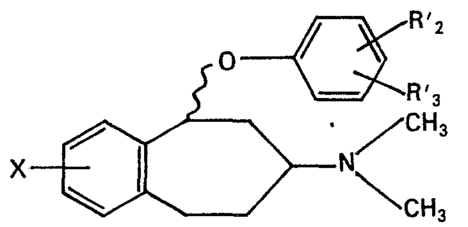

($I_A$)

dans laquelle $R'_2$, $R'_3$ et X ont la signification déjà indiquée et que,

- soit, à condition d'avoir préparé un produit de formule ($I_A$) dans laquelle l'un au moins de $R'_2$ ou de $R'_3$ représente un radical nitro, on réduit ledit produit de formule ($I_A$) pour obtenir un produit de formule:

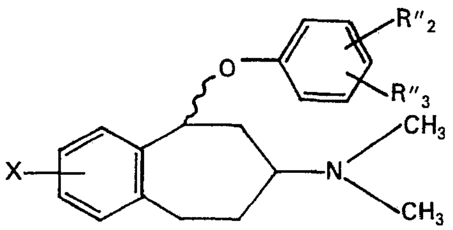

($I_B$)

dans laquelle X a la signification déjà indiquée, l'un au moins de $R''_2$ ou de $R''_3$ représente un radical amino et l'autre substituant $R''_2$ ou $R''_3$ a la signification de $R_2$,

- soit, à condition d'avoir préparé un produit de formule ($I_A$) dans laquelle $R'_2$ représente un atome de brome et $R'_3$ représente un atome d'hydrogène, on hydrogénolyse ledit produit de formule ($I_A$) pour obtenir un produit de formule:

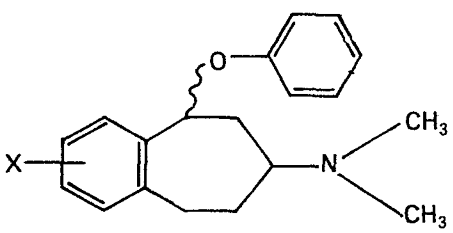

($I_C$)

dans laquelle X a la signification déjà indiquée, et que, soit on isole et, si désiré, salifie les produits de formules ($I_A$), ($I_B$), ($I_C$) ainsi obtenus, correspondant à des produits de formule (I') dans laquelle $R_1$ représente un radical méthyle, soit enfin on désalcoyle ces derniers et, le cas échéant, salifie les produits de formule (I') ainsi obtenus dans laquelle X, $R_2$ et $R_3$ ont la signification déjà indiquée et $R_1$ représente un atome d'hydrogène.

8. Procédé selon la revendication 7, caractérisé en ce que l'on utilise au départ un composé de formule II dans laquelle X représente un atome d'hydrogène.

9. Médicaments, caractérisés en ce qu'ils sont constitués par l'un au moins des dérivés de formule I' telle que définie à la revendication 1, ou par l'un au moins de leurs sels pharmaceutiquement acceptables.

10. Médicaments, caractérisés en ce qu'ils sont constitués par l'un au moins des dérivés de formule I telle que définie à la revendication 2 ou par l'un au moins de leurs sels pharmaceutiquement acceptables.

11. Médicaments, caractérisés en ce qu'ils sont constitués par l'un au moins des dérivés de formule I, telle que définie à l'une quelconque des revendications 3 ou 4 ou par l'un au moins de leurs sels pharmaceutiquement acceptables.

12. Médicaments, caractérisés en ce qu'ils sont constitués par l'un au moins des dérivés de formule I, tels que définis à la revendication 5 ou 6, ou par l'un au moins de leurs sels pharmaceutiquement acceptables.

13. Compositions pharmaceutiques, caractérisées en ce qu'elles renferment, à titre de principe actif, l'un au moins des médicaments, tels que définis à l'une des revendications 9, 10, 11 ou 12.

**Claims**

1. Derivatives of 5H-benzocyclohepten-7-amine as well as their addition salts with the mineral or organic acids, characterised in that they correspond to the general formula:

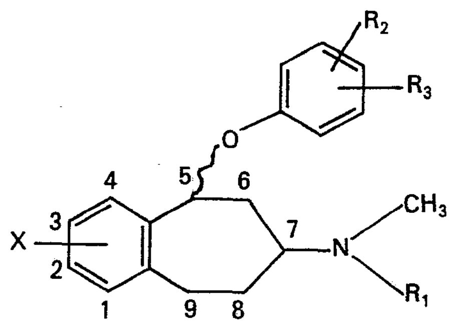

(I')

in which $R_1$ represents a hydrogen atom or a methyl radical, $R_2$ and $R_3$, being the same or different, represent a hydrogen atom, a fluorine, chlorine or bromine atom, or a nitro, amino, trifluoromethyl or methoxy radical and X represents a hydrogen or halogen atom.

2. Derivatives according to Claim 1 as well as their addition salts with the mineral or organic acids, characterised in that they correspond to the general formula:

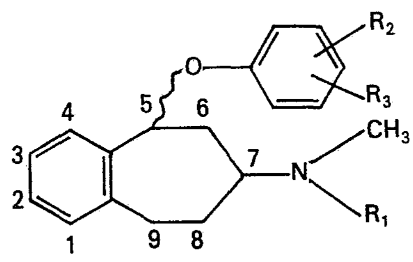

(I)

in which $R_1$, $R_2$ and $R_3$ are defined as in Claim 1.

3. Derivatives corresponding to the general formula (I) of Claim 2 as well as their addition salts with the mineral or organic acids, characterised

in that in the said formula (I) $R_1$ represents a methyl radical and $R_2$ and $R_3$ have the meaning already indicated in Claim 1.

4. Derivatives corresponding to the general formula (I) of Claim 2 as well as their addition salts with the mineral or organic acids, characterised in that in the said formula (I) $R_1$ represents a methyl radical and one at least of $R_2$ and $R_3$ represents a hydrogen atom.

5. Any one of the derivatives corresponding to the formula (I) of which the names are as follows:
- the cis and trans isomers of N,N-dimethyl 5-[(4-nitrophenyl)oxy] 6,7,8,9-tetrahydro 5H-benzocyclohepten 7-amine,
- the cis isomer of N,N-dimethyl 6,7,8,9-tetrahydro 5-[(4-(trifluoromethyl) phenyl]oxy] 5H-benzocyclohepten 7-amine,
- the cis isomer of N,N-dimethyl 5-[[4-bromophenyl]oxy] 6,7,8,9-tetrahydro 5H-benzocyclohepten 7-amine, as well as their addition salts with the mineral or organic acids.

6. N,N-dimethyl 5-[(4-nitrophenyl)oxy] 6,7,8,9-tetrahydro 5H-benzocyclohepten 7-amine fumarate (cis isomer).

7. Process for preparing the derivatives, as well as their salts, as defined by the formula I′ of claim 1, the said process being characterised in that a product of formula:

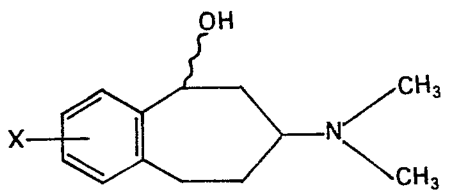

(II)

in which X represents a hydrogen or halogen atom, is reacted with a product of formula:

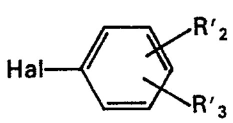

(III)

in which Hal represents a fluorine, chlorine or bromine atom, $R'_2$ represents a fluorine, chlorine or bromine atom or a nitro, trifluoromethyl or methoxy radical and $R'_3$ represents a hydrogen atom or $R'_2$, to obtain a product of formula:

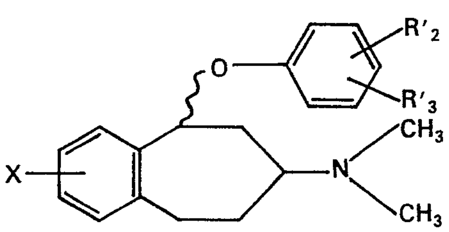

($I_A$)

in which $R'_2$, $R'_3$ and X have the meaning already indicated, and in that
- either, providing a product of formula ($I_A$) has been prepared in which one at least of $R'_2$ or $R'_3$ represents a nitro radical, the said product of formula ($I_A$) is reduced to obtain a product of formula:

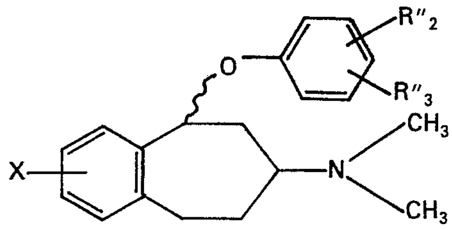

($I_B$)

in which X has the meaning already indicated, one at least of $R''_2$ or $R''_3$ represents an amino radical and the other substituent $R''_2$ or $R''_3$ has the meaning of $R_2$,
- or, providing a product of formula($I_A$) has been prepared in which $R'_2$ represents a bromine atom and $R'_3$ represents a hydrogen atom, the said product of formula ($I_A$) is hydrogenolysed to obtain a product of formula:

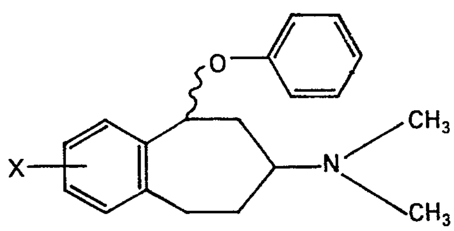

($I_C$)

in which X has the meaning already indicated, and in that either the products of formulae ($I_A$), ($I_B$) and ($I_C$) thus obtained, corresponding to products of formula (I′) in which $R_1$ represents a methyl radical, are isolated and, if desired, salified, or finally the latter are dealkylated and, if need be, the products of formula (I′) thus obtained are salified in which formula X, $R_2$ and $R_3$ have the meaning already indicated and $R_1$ represents a hydrogen atom.

8. Process according to Claim 7, characterised in that, to start, a compound of formula II is used in which X represents a hydrogen atom.

9. Medicaments, characterised in that they are constituted by one at least of the derivatives of formula I′ as defined in Claim 1 or by one at least of their pharmaceutically-acceptable salts.

10. Medicaments, characterised in that they are constituted by one at least of the derivatives of formula I as defined in Claim 2 or by one at least of their pharmaceutically-acceptable salts.

11. Medicaments, characterised in that they are constituted by one at least of the derivatives of formula I as defined in any one of the Claims 3 or 4 or by one at least of their pharmaceutically-acceptable salts.

12. Medicaments, characterised in that they are constituted by one at least of the derivatives of formula I as defined in Claim 5 or 6 or by one at least of their pharmaceutically-acceptable salts.

13. Pharmaceutical compositions, characterised in that they contain, as active principle, one at least of the medicaments, as defined in one of the Claims 9, 10, 11 or 12.

**Patentansprüche**

1. 5H-Benzocyclohepten-7-amin-Derivate sowie deren Additionssalze mit Mineral- oder organischen Säuren, dadurch gekennzeichnet, dass sie die allgemeine Formel:

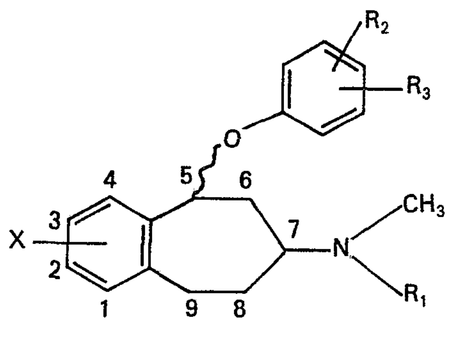

(I')

besitzen, in der
$R_1$ ein Wasserstoffatom oder einen Methylrest bedeutet,
$R_2$ und $R_3$, die gleich oder verschieden sein können, ein Wasserstoff-, Fluor-, Chlor- oder Bromatom, einen Nitro-, Amino-, Trifluormethyl- oder Methoxyrest bedeuten und
X ein Wasserstoff- oder Halogenatom bedeutet.

2. Derivate gemäss Anspruch 1 sowie deren Additionssalze mit Mineral- oder organischen Säuren, dadurch gekennzeichnet, dass sie der allgemeinen Formel:

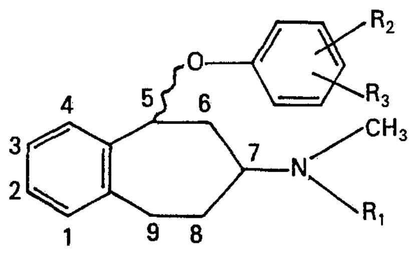

(I)

entsprechen, in der $R_1$, $R_2$ und $R_3$ wie in Anspruch 1 definiert sind.

3. Derivate der allgemeinen Formel (I) gemäss Anspruch 2 sowie deren Additionssalze mit Mineral- oder organischen Säuren, dadurch gekennzeichnet, dass in der Formel (I) $R_1$ einen Methylrest bedeutet und $R_2$ und $R_3$ die in Anspruch 1 angegebene Bedeutung besitzen.

4. Derivate der allgemeinen Formel (I) gemäss Anspruch 2 sowie deren Additionssalze mit Mineral- oder organischen Säuren, dadurch gekennzeichnet, dass in der Formel (I) $R_1$ einen Methylrest bedeutet und zumindest einer der Reste $R_2$ und $R_3$ ein Wasserstoffatom bedeutet.

5. Derivate der Formel (I) mit den folgenden Bezeichnungen:
die cis- und trans-Isomeren von N,N-Dimethyl-5-[(4-nitrophenyl)-oxy]-6,7,8,9-tetrahydro-5H-benzocyclohepten-7-amin, das cis-Isomere von N,N-Dimethyl-6,7,8,9-tetrahydro-5-[[4-(trifluormethyl)-phenyl]oxy]-5H-benzocyclo hepten-7-amin,
das cis-Isomere von N,N-Dimethyl-5-[(4-bromphenyl)-oxy]-6,7,8,9-tetrahydro-5H-benzocyclohepten-7-amin sowie deren Additionssalze mit Mineral- oder organischen Säuren.

6. N,N-Dimethyl-5-[(4-nitrophenyl)-oxy]-6,7,8,9-tetrahydro-5H-benzocyclohepten-7-amin--fumarat (cis-Isomeres).

7. Verfahren zur Herstellung von Derivaten sowie deren Salzen wie durch die Formel I' gemäss Anspruch 1 definiert, dadurch gekennzeichnet, dass man ein Produkt der Formel:

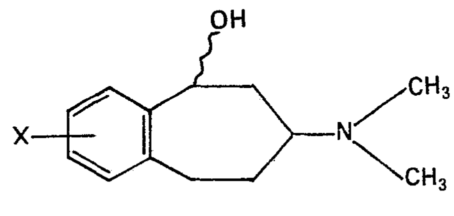

(II)

in der X ein Wasserstoff- oder Halogenatom bedeutet, mit einem Produkt der Formel:

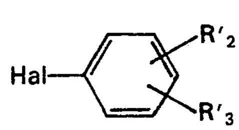

(III)

umsetzt, in der Hal ein Fluor-, Chlor- oder Bromatom bedeutet, $R'_2$ ein Fluor-, Chlor- oder Bromatom, einen Nitro-, Trifluormethyl- oder Methoxyrest bedeutet und $R'_3$ ein Wasserstoffatom oder $R'_2$ bedeutet, um ein Produkt der Formel:

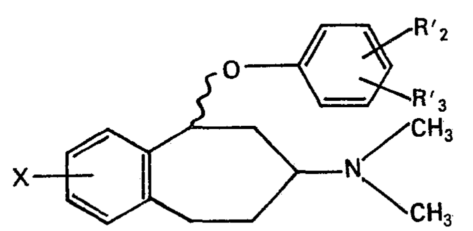

($I_A$)

zu erhalten, in der $R'_2$, $R'_3$ und X die angegebene Bedeutung besitzen, und dass man
entweder, wenn man ein Produkt der Formel ($I_A$) hergestellt hat, in der zumindest einer der Reste $R'_2$ oder $R'_3$ einen Nitrorest bedeutet, dieses Produkt der Formel ($I_A$) reduziert, um ein Produkt der Formel:

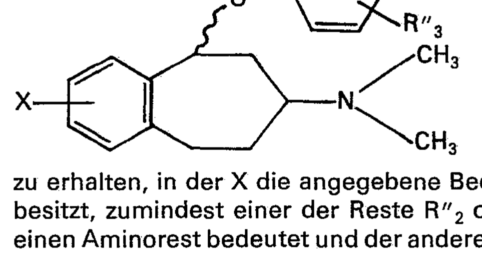

($I_B$)

zu erhalten, in der X die angegebene Bedeutung besitzt, zumindest einer der Reste $R''_2$ oder $R''_3$ einen Aminorest bedeutet und der andere Substituent $R''_2$ oder $R''_3$ die Bedeutung von $R_2$ besitzt, oder, wenn man ein Produkt der Formel ($I_A$) hergestellt hat, worin $R'_2$ ein Bromatom bedeutet

und $R'_3$ ein Wasserstoffatom bedeutet, dieses Produkt der Formel ($I_A$) einer Hydrogenolyse unterzieht, um ein Produkt der Formel:

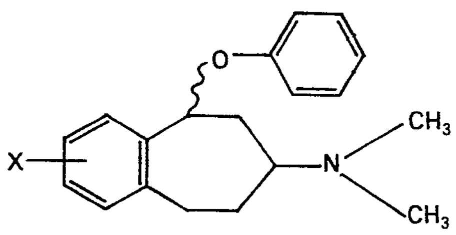

($I_C$)

zu erhalten, in der X die angegebene Bedeutung besitzt,
und dass man entweder die so erhaltenen Produkte der Formel ($I_A$), ($I_B$) und ($I_C$) entsprechend Produkten der Formel (I'), in der $R_1$ einen Methylrest bedeutet, isoliert und gewünschtenfalls in ein Salz überführt oder schliesslich diese letzteren dealkyliert und gegebenenfalls die so erhaltenen Produkte der Formel (I'), in der X, $R_2$ und $R_3$die angegebene Bedeutung besitzen und $R_1$ ein Wasserstoffatom darstellt, in ein Salz überführt.

8. Verfahren gemäss Anspruch 7, dadurch gekennzeichnet, dass man als Ausgangssubstanz eine Verbindung der Formel II verwendet, in der X ein Wasserstoffatom bedeutet.

9. Arzneimittel, dadurch gekennzeichnet, dass sie zumindest aus einem der Derivate der Formel (I') gemäss Anspruch 1 oder zumindest einem ihrer pharmazeutisch verträglichen Salze bestehen.

10. Arzneimittel, dadurch gekennzeichnet, dass sie aus zumindest einem der Derivate der Formel (I) gemäss Anspruch 2 oder aus einem ihrer pharmazeutisch verträglichen Salze bestehen.

11. Arzneimittel, dadurch gekennzeichnet, dass sie aus zumindest einem der Derivate der Formel (I) wie in einem der Ansprüche 3 oder 4 definiert oder aus einem ihrer pharmazeutisch verträglichen Salze bestehen.

12. Arzneimittel, dadurch gekennzeichnet, dass sie aus zumindest einem der Derivate der Formel (I) wie in Anspruch 5 oder 6 definiert oder aus zumindest einem ihrer pharmazeutisch verträglichen Salze bestehen.

13. Pharmazeutische Zusammensetzungen, dadurch gekennzeichnet, dass sie als Wirkstoff zumindest eines der Arzneimittel gemäss einem der Ansprüche 9, 10, 11 oder 12 enthalten.